# EUROPEAN PATENT SPECIFICATION

(11) **EP 4 569 388 B1**
(45) Date of publication and mention of the grant of the patent: **11.11.2026**
(21) Application number: 23754172.7
(22) Date of filing: 03.08.2023
(51) Int. Cl.: G05B 23/02

(54) **TRACKING PROGRESS OF PROACTIVE MONITORING ACTIONS TO AVOID DOWNTIME OR DEGRADED PERFORMANCE OF MEDICAL DEVICES**
VERFOLGUNG DES FORTSCHRITTS VON PROAKTIVEN ÜBERWACHUNGSAKTIONEN ZUR VERMEIDUNG VON AUSFALLZEIT ODER DEGRADIERTER LEISTUNG VON MEDIZINISCHEN VORRICHTUNGEN
SUIVI DE LA PROGRESSION D'ACTIONS DE SURVEILLANCE PROACTIVES POUR ÉVITER L'ARRÊT OU DES PERFORMANCES DÉGRADÉES DE DISPOSITIFS MÉDICAUX

(30) Priority: 10.08.2022 US 202263396695 P
(43) Date of publication of application: 18.06.2025
(73) Proprietor: Koninklijke Philips N.V., 5656 AE Eindhoven (NL)
(72) Inventor: KORST, Johannes Henricus Maria, 5656 AG Eindhoven (NL); PRONK, Serverius Petrus Paulus, 5656 AG Eindhoven (NL); SREENIVASAN, Rithesh, 5656 AG Eindhoven (NL); BARBIERI, Mauro, 5656 AG Eindhoven (NL); PETERS, Marc André, 5656AG Eindhoven (NL)
(74) Representative: Philips Intellectual Property & Standards
(86) International application number: PCT/EP2023/071484
(87) International publication number: WO 2024/033196

(56) References cited:
- WO-A1-2022/033988
- US-A1- 2006 149 837

## Description

### FIELD

The following relates generally to the medical device maintenance arts, medical imaging device maintenance arts, medical device maintenance scheduling arts, and related arts.

### BACKGROUND

To proactively avoid unplanned downtime, medical imaging systems, such as magnetic resonance imaging (MRI), a computed tomography (CT), a positron emission tomography (PET), interventional radiology (IR), X-ray, and image-guided therapy (IGT) or other medical imaging systems, as well as picture archiving and communication systems (PACS) are remotely monitored. These systems produce log messages (or alerts) that can be displayed on dashboards that are used by remote monitoring engineers to recognize issues before they result in downtime or degraded performance. In addition, remote monitoring engineers can react on alerts generated by predictive models that use multiple log messages, possibly requiring additional precedence and timing constraints.

For example, log messages can indicate that a filament of an X-ray tube is nearing its end of life and that a new X-ray tube can already by ordered to be able to quickly replace the tube whenever it breaks or to proactively replace the tube before it is broken. Similarly, high central processing unit (CPU) load on specific parts of a PACS can indicate that some software parts unexpectedly suffer from memory leakage leading to excessive memory and CPU usage and a remote restart of these parts can avoid subsequent downtime or degraded performance.

In some cases, the remote monitoring can be completely automated. In those cases, the log messages provide sufficient information to determine the precise root cause of issues. An issue as well as its correct root cause can be derived from specific patterns of log messages in these cases. In other cases, determining the correct root cause may be difficult or even impossible to automate. This may be due to the fact that additional checks need to be performed to determine the correct root cause. Performing some of these checks may only be possible when a human is remotely logged in. Completely automating these steps may not be feasible or desirable. Developing an automatic process for a complete resolution tree may be considered too costly, especially if it concerns issues that rarely occur. Furthermore, the required automated remote login may be not desired by the hospitals that are using the systems, for reasons of security. Also, the additional checks may interfere with the normal functioning of such a system, so that they may only be carried out at times when no patients are being examined. This requires coordination between the remote monitoring engineers and the people that operate the systems in the hospital to identify a suitable moment for the additional checks to be carried out.

In the latter cases, resolving issues, which are identified either automatically by recognizing suspicious patterns in the log messages or by the remote monitoring engineers based on their own experience, can be a complex process. At a single moment in time, there can be multiple issues that need to be resolved and each of them might require remote login at different times that are suitable for the respective hospitals. Consequently, it may get unnoticed when some of the identified issues are left open for further analysis leading to unneeded down time or degraded performance.

The following discloses certain improvements to overcome these problems and others.

A document WO2022/033988A1 discloses a non-transitory computer readable medium that stores instructions executable by at least one electronic processor to perform a method of generating a recommendation engine for recommending actions during performance of a fault-finding task. The method comprises: converting a collection of historical fault-finding process sequences into a fault-finding tree having nodes, action edges and outcome edges connecting the nodes. The action edges are labeled with actions of the historical fault-finding process sequences and the outcome edges are labeled with outcomes of the historical fault-finding process sequences. The nodes include terminal nodes labeled with root causes or solutions identified by the historical fault-finding process sequences. A visualization of the fault- finding tree is provided on a user interface on a service device operable by a field service engineer.

A document US2006/149837A1 discloses a technique for automatically prioritizing requests for service of systems. A service request is generated upon detection of an indicator of a potentially serviceable condition in the system. Based upon the service request, a knowledge base is consulted that may contain prioritization scores for particular indicators, conditions, systems, customers and so forth. Service recommendations are then prioritized based upon the information. Similar prioritization among service requests may also be made based upon the information. The prioritizations may be adjusted or new prioritizations may be made based upon other factors, such as downtime experienced or anticipated, customer need, contract provisions, and so forth.

### SUMMARY

In one aspect, a non-transitory computer readable medium is disclosed, as defined in claim 1.

In another aspect, a method is disclosed, as defined in claim 11.

One advantage resides in determining a correct root cause of a maintenance issue with a medical device.

Another advantage resides in minimizing an amount of time spent to resolve an issue with a medical device.

Another advantage resides in reducing security concerns related to data for performing service actions on a medical device.

Another advantage resides in minimizing an amount of time spent to resolve an issue with a medical device by distributing tasks to multiple remote monitoring engineers.

A given embodiment may provide none, one, two, more, or all of the foregoing advantages, and/or may provide other advantages as will become apparent to one of ordinary skill in the art upon reading and understanding the present disclosure.

### BRIEF DESCRIPTION OF THE DRAWINGS

The disclosure may take form in various components and arrangements of components, and in various steps and arrangements of steps. The drawings are only for purposes of illustrating the preferred embodiments and are not to be construed as limiting the disclosure.
FIGURE 1 diagrammatically illustrates an illustrative system for determining a root cause and at least one recommended service action for servicing a medical device in accordance with the present disclosure.
FIGURE 2 shows exemplary flow chart operations of the system of FIGURE 1.

### DETAILED DESCRIPTION

In medical imaging servicing, a remote service engineer (RSE; or proactive monitoring engineer or the like) handles issues opened manually or by a predictive algorithm. However, resolving an open issue may involve multiple steps, with some steps being time-constrained by factors such as needing to log into the imaging system at a time when it is not being used for patient imaging. Issues may therefore be forgotten, and remain unresolved until they manifest as a device failure or other detrimental way.

The following discloses a smart ticketing system for issues. The system defines an issue as a pattern of observations, such as AΛBΛNOT(C) where A and B and C may be log messages, for example. Each such defined issue is associated to a resolution tree, which is typically constructed by experts and includes a root node corresponding to the issue, intermediate nodes representing service actions carried out to resolve the issue, and leaf nodes corresponding to possible diagnosed root causes. Each leaf node has an associated probability of the root cause and severity of that root cause.

The system monitors results of service actions performed to diagnose the issue as they are entered into the system by the RSE, and dynamically updates the probabilities of the various root causes as certain leaves become unreachable based on these results. Scheduled but not-yet-performed service actions are also logged.

The system may also compute the severity values of the root causes corresponding to the leaf nodes based on historical data, and such updating may optionally also dynamically update the computed severities of the root causes corresponding to the leaf nodes as historical data continually accrues. Additionally, the system computes an aggregate probability and/or severity for each issue as a sum over all leaf nodes of the associated resolution tree of the product of the probability and severity.

The system further provides a user interface (UI) to enable the RSE to access this information. In some examples, a two-level UI can be provided. The first level is an overview screen listing all open issues with information such as the aggregate severity and top-K most likely root causes. The second level is reached by clicking on a specific issue which brings up additional information for that issue.

In some embodiments, the disclosed system provides time-based reminders of open issues. This feature takes into account scheduled service actions, so that for example the RSE does not receive a reminder if a next service action is already scheduled.

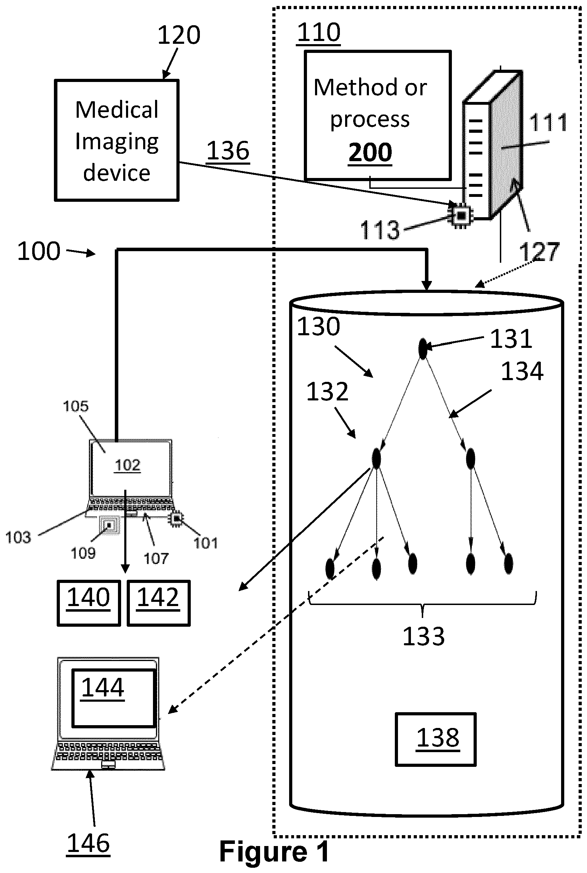

With reference to FIGURE 1, an illustrative servicing support system **100** for supporting a service engineer in servicing a device **120** (e.g., a medical imaging device - also referred to as a medical device, an imaging device, imaging scanner, and variants thereof) is diagrammatically shown. By way of some non-limiting illustrative examples, the medical imaging device under service may be a magnetic resonance imaging (MRI) scanner, a computed tomography (CT) scanner, a positron emission tomography (PET) scanner, a gamma camera for performing single photon emission computed tomography (SPECT), an interventional radiology (IR) device, or so forth. (More generally, the disclosed approach can be applied in conjunction with any type of computerized device that automatically generates log data that are analyzed by predictive models to predict component failures, e.g., the approach could be applied to a commercial airliner, radiation therapy device, or so forth). As shown in FIGURE 1, the servicing support system **100** includes, or is accessible by, a service device **102** that may for example be a workstation or electronic processing device used by an RSE. The service device **102** may for example be a portable device such as a notebook computer that is carried or accessed by an RSE. The service device **102** can be a desktop computer or a personal device, such as a mobile computer system such as a laptop or smart device. In other embodiments, the service device **102** may be an imaging system controller or computer integral with or operatively connected with the imaging device undergoing service (e.g., at a medical facility). As another example, the service device **102** may be a portable computer (e.g., notebook computer, tablet computer, or so forth) carried by a RSE performing diagnosis of a fault with the imaging device and ordering of parts. In another example, the service device **102** may be the controller computer of the imaging device under service, or a computer based at the hospital. In other embodiments, the service device may be a mobile device such as a cellular telephone (cellphone) or tablet computer.

The service device **102** includes a display **105** via which alerts generated by predictive failure models are displayed, along with likely root cause and service action recommendation information as disclosed herein. The service device **102** also preferably allows the service engineer to interact with the servicing support system via at least one user input device **103** such a mouse, keyboard, or touchscreen. The service device further includes an electronic processer **101** and non-transitory storage medium **107** (internal components which are diagrammatically indicated in FIGURE 1). The non-transitory storage medium **107** stores instructions which are readable and executable by the electronic processor **101** for interfacing with the servicing support system **100.** The service device **102** also includes a communication interface **109** to communicate with a backend server or processing device **111,** which typically implements the computational aspects of the servicing support system **100** (e.g., the server **111** has the processing power for implementing computationally complex aspects of the servicing support system **100**). Such communication interfaces **109** include, for example, a wired and/or wireless Ethernet interface (e.g., in the case in which the service device **102** is a RSE workstation); or in the case in which the service device **102** is a portable FSE device the interface **109** may be a wireless Wi-Fi or 4G/5G interface or the like for connection to the Internet and/or an intranet. Some aspects of the servicing support system **100** may also be implemented by cloud processing or other remote processing (that is, the server computer **111** may be embodied as a cloud-based computing resource comprising a plurality of interconnected servers).

In illustrative FIGURE 1, the servicing support system further includes a backend **110** (e.g., implemented and/or owned by the imaging device vendor or leased by the vendor from a cloud computing service provider). The backend **110** receives log data (e.g., a machine log automatically generated by the medical imaging device **120,** a service log for the medical imaging device **120,** and/or so forth) on a continuous or occasional basis (e.g., in some setups the imaging device **120** uploads machine log entries to the backend **110** on a daily basis). The backend may optionally perform processing for performing predictive fault modeling, in which case an issue may be opened automatically (e.g., if fault modeling predicts an X-ray tube will likely fail in the next month, a service ticket may be automatically generated to replace the X-ray tube). The backend may optionally additionally or alternatively provide a ticketing system or the like via which users can manually open a ticket for an open issue.

The backend further performs maintenance service analyses on the backend server **111,** which is equipped with an electronic processor **113** (diagrammatically indicated internal component). These analyses provide automated tracking of the handling of an automatically or manually opened issue. The server **111** is equipped with non-transitory storage medium **127** (internal components which are diagrammatically indicated in FIGURE 1). While a single server computer is shown, it will be appreciated that the backend **110** may more generally be implemented on a single server computer, or a server cluster, or a cloud computing resource comprising ad hoc-interconnected server computers, or so forth. Furthermore, while FIGURE 1 shows a single medical imaging device **120,** more generally the database backend **110** will receive log data from many medical imaging devices (e.g., tens, hundreds, or more imaging devices) and performs the disclosed processing for each such medical imaging device.

With continuing reference to FIGURE 1, the non-transitory computer readable medium **127** stores a resolution tree **130** related to resolution of a maintenance issue of the medical imaging device **120.** As shown in FIGURE 1 which presents a rendering of the resolution tree **130,** the resolution tree **130** includes leaf nodes **132** (depicted as circles) and edges **134** (depicted as arrows) connecting the nodes. The resolution tree **130** contains data related to maintenance issues for the medical imaging device **120,** and nodes **132** correspond to possible root causes of the maintenance issue. Each leaf node **132** includes a probability of the corresponding root cause. In some embodiments, each leaf node **132** further includes a severity of the corresponding root cause. The severity of a corresponding root cause can be computed on the basis of various metrics, such as cost in monetary units, cost in terms of downtime, medical imaging device performance, and/or cost as measured by another chosen severity metric. In one example, the severity of a root cause represents an impact of the root cause on a performance of the medical imaging device, for example due to degraded image quality and/or inability to perform certain type(s) of imaging and/or diagnostic clinical tasks due to the root cause. In another example, the severity of a root cause represents a downtime of the medical imaging device caused by the root cause. In another example, the severity of a root cause represents an aggregation of the impact on performance of the medical imaging device and downtime of the medical imaging device caused by the root cause. In another example, the severity of a root cause represents a monetary cost of resolving the root cause. In some embodiments, the resolution tree **130** further includes a root node **131** corresponding to the maintenance issue, and a plurality of intermediate nodes **133** each representing service actions which can be carried out attempting to resolve the maintenance issue. While a single illustrative resolution tree **130** is shown by way of illustrative example, there may be a plurality of resolution trees stored, with each tree constructed for a specific issue or class or type or category of issues.

The non-transitory storage medium **127** further stores instructions executable by the electronic processor **113** of the backend server **111** to perform a method **200** of assigning tasks to different RSEs for a current service case for maintenance of the medical imaging device **120.**

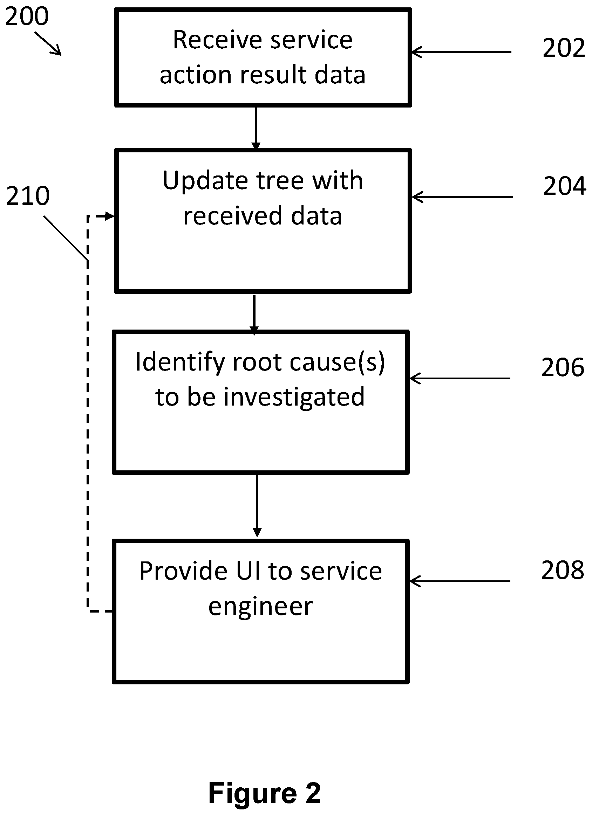

With continuing reference to FIGURE 1 and further reference to FIGURE 2, an illustrative embodiment of the method **200** executable by the electronic processor **113** is diagrammatically shown as a flowchart. In some examples, the method **200** may be performed at least in part by cloud processing.

To begin the method **200,** at an operation **202,** data **136** related to results of a completed service action performed to diagnose the maintenance issue is received, for example, by the backend server **111** (operable by the vendor) from the customer of the medical device **120.** (It is assumed here that the completed service action is addressing an existing open issue).

At an operation **204,** the resolution tree **130** is updated with the received data including updating the probabilities of the root causes of the corresponding leaf nodes **132.** In some embodiments, the updating operation **204** also includes computing or updating severity values based on historical data of historical service actions that were successful in resolving previous maintenance issues, and updating the resolution tree **130** with the computed severity values. For example, computing or updating the severity values includes computing an aggregate severity for the maintenance issue as a sum over all leaf nodes **132** as the product of the computed probability and severity values, and issuing an alert on the service device **102** regarding the maintenance issue if the aggregate severity exceeds a threshold. As an example of such updating, consider a resolution tree **130** with three leaf nodes A, B, C, with leaf node A labeled with probability P_{A}=0.50 and severity 1, leaf node B labeled with probability P_{B}=0.30 and severity 2, and leaf node C labeled with probability P_{C}=0.20 and severity 3. If the completed service action has eliminated leaf node C (for example, the completed service action produces a result which excludes the root cause associated with leaf node C), then P_{C}=0 is set, and the probabilities P_{A} and P_{B} of the respective remaining leaf nodes A and B can be adjusted accordingly, for example by proportionally distributing the (now-eliminated) probability P_{C}=0.20 among the remaining nodes A and B. For example, this can be done by setting ${P}_{A} = 0.50 + \frac{0.50}{0.50 + 0.30} \times \left(0.20\right) = 0.625$ and ${P}_{B} = 0.30 + \frac{0.30}{0.50 + 0.30} \times \left(0.20\right) = 0.375$ so that P_{A} + P_{B} + P_{C} = 0.625 + 0.375 + 0 = 1.000 still holds.

At an operation **206,** one or more root causes to be investigated are identified based at least on the updated probabilities of the root causes of the corresponding leaf nodes **132.** In the previous example, of the two remaining leaf nodes A and B, node A has highest probability (adjusted P_{A}=0.625) and hence this tends toward investigating the possibility of the root node associated with node A next. In some examples, the one or more root causes to be investigated are identified further based on the severities of the corresponding root causes. In the ongoing example, this would tend toward investigating the root cause of node B next, since it has higher severity 2 compared with the severity 1 of node A (here higher value is assumed to be higher severity, i.e. higher cost in monetary units, downtime, or another chosen severity metric). A weighted priority can be employed in some embodiments, e.g. choosing the next leaf node/root cause to investigate based on a weighted combination of the probabilities and severities. In some embodiments, the one or more service actions to be performed can be identified based on the one or more root causes to be investigated and the intermediate nodes **133.** To do so, the back end server is configured to receive a schedule **138** of scheduled service actions from a scheduler, and output a reminder on the service device **102** to perform or schedule one or more of the service actions to be performed that are not scheduled service actions.

At an operation **208,** the one or more root causes to be investigated and the updated probabilities of the respective one or more root causes to be investigated (and optionally the severities of the respective one or more root causes to be investigated) are displayed on the display device **105** of the service device **102.** To do so, a first user interface (UI) **140** can be provided on the display device **105,** and be configured to show the computed aggregate severity for the maintenance issue. A second UI **142** can also be provided on the display device **105,** and be configured to show information related to a specific root cause of the maintenance issue. In some examples, the second UI **142** can be displayed upon receiving a user input via the at least one user input device **103**) indicative of a selection of a portion of the first UI **140.**

In some embodiments, the receive operation **202,** the update operation **204,** and the display operation **208** can be repeated (as indicated by an arrow **210** in FIGURE 2) for each successive completed service action to update the displayed one or more possible root causes to be investigated and the probabilities of the respective one or more root causes to be investigated.

In some embodiments, the resolution tree **103** and/or the one or more root causes to be investigated and the updated probabilities of the respective one or more root causes to be investigated (and optionally the severities of the respective one or more root causes to be investigated) are displayed on a customer UI **144** displayed on a customer electronic processing device **146** operable by the customer.

### EXAMPLES

The servicing support system **100** is configured to automatically track the proactive monitoring process that is carried out by the remote monitoring engineers. This involves estimating the severity of each of the identified issues and monitoring whether sufficient progress is being made in resolving the open issues, considering their estimated severity. And in case insufficient progress is being detected - especially for issues that are assumed to have a high severity - dedicated alerts are generated that bring these issues to the attention of the remote service engineers. Furthermore, the proposed monitoring can help the remote monitoring engineers to determine which of the issues should be considered next for further resolution. The order in which the issues are to be handled next will repeatedly be adapted whenever there is new information on the probability and severity of the possible root causes that remain open.

Let an issue be specified by a logical pattern of the occurrence of different types of log messages that may involve additional precedence and timing conditions on these log messages. For example, a logical pattern may be the occurrence of log messages A and B, where A must be observed before B and where the time between observing A and B may not be larger than 5 minutes, while in the interval between observing A and B, there is no occurrence of a third log message C. This pattern could be described as *A* Λ *B* Λ *NOT* (*C*) having additional precedence and timing conditions. Alternatively, an issue can be specified by the fact that a given parameter that is being monitored exceeds a certain threshold. For example, that the number of files that reside in a given waiting queue exceeds a given threshold. Or, alternatively, that the CPU usage of a given process exceeds a certain threshold, when measured over a past time unit, where the time unit is an appropriately selected measure of time, such as 5 minutes or 1 hour.

With each issue *i*, a resolution tree *Tᵢ* is associated. The resolution tree *Tᵢ* = (*V, E*) is a directed rooted tree with root node *r* and multiple leaf nodes *l*_{*i*,1}, *l*_{*i*,2}, ..., *l*_{*i,n*(*i*)}, where *n*(*i*) denotes the number of different root causes that can be associated with issue i. All edges in *Tᵢ* are directed from the root node *r* towards the leaf nodes. Each leaf node *l_{i,j}* corresponds to a specific root cause having associated probability *p*(*l_{i,j}*), severity *s*(*l_{i,j}*), and a sequence *A*(*l_{i,j}*) of service actions that will solve the issue given that the root cause is given by *l_{i,j}.* The intermediate nodes are considered to be checks that a remote monitoring engineer has to carry out and possibly require to remotely login into the system at risk and potentially require making an appointment for doing the remote login at a moment in time that the system is not being used.

Let the severity *s*(*l_{i,j}*) of the root cause associated with leaf node *l_{i,j}* be given by a real number in [0,1], where a value close to 0 indicates a low severity and a value close to 1 a high severity. A high severity indicates that the system will experience downtime if the issue is not handled appropriately. Furthermore, the probability *p*(*l_{i,j}*) gives the estimated probability that the root cause of issue *i* is the one associated with leaf node *l_{i,j},* so that 0 ≤ *p*(*l_{i,j}*) ≤ 1 for all *j* ∈ {1,2, ..., *n*(*i*)} and according to Equation (1): ${\sum }_{j = 1}^{n \left(i\right)} p \left({l}_{i , j}\right) = 1$ (1)

Initially, if no additional checks have been performed yet on resolving issue *i*, then the severity *s*(*i*) of issue *i* can be estimated by a weighted average of the severities of all its leaf nodes. For example, Equation (2) states: $s \left(i\right) = {\sum }_{j = 1}^{n \left(i\right)} p \left({l}_{i , j}\right) ⋅ s \left({l}_{i , j}\right) .$ (2)

After new information has become available as a result of an additional check, some leaf nodes are no longer reachable. Updating the severity of the issue can be realized by setting the probability of these leaf nodes to zero and by proportionally increasing the probability of the other leaf nodes such that the sum of their probabilities again sum up to 1. Alternatively, the new information might change the relative probabilities of the remaining root causes, resulting in an alternative adaptation of the probabilities of the remaining root causes so that they add up to 1 again. Estimates of the probabilities and severities are based on data of historical issues, as far as available for the given information that specifies the issue. The more information is available on similar previous issues, the more accurate these estimates will be. Initially, if no information is available, all potential root causes are considered equally probable and equally severe. And, when increased data is gathered on historical issues, these prior probabilities are adjusted accordingly.

The definition of severity per issue can be used to rank the issues to determine the issue that should be considered next for progressing its resolution. In other words, the estimated severities could be used to give advice to the service engineers on which issue to work on next. For this advice, one can also take into account the appointments that have already been made for the remote logins. For example, if there is an appointment for a remote login scheduled in the next 10 minutes, then the advice could take this into account by not advising to consider working on a next step for an open issue if this would take more than the time available before this appointment. However, in the first place, keeping track of the changing estimated severities are intended to be used for tracking the progress of solving the various issues.

The progress of the resolution of the open issues should be captured, for example, in a UI that the service engineer uses to input the service actions that have been taken. The service engineers record which service actions have been carried out for each of the issues. An issue will be identified by a case or ticket number and the type of warning or error log message or alert, with associated data, will allow the monitoring application to determine the type of issue at hand and identify the corresponding resolution tree with associated additional checks (represented by the intermediate nodes) and the root causes (represented by the leaf nodes).

The UI is assumed to consist of one main window, showing an ordered list of open issues, with the possibility to order the open issues according to different criteria as given in Table 1.

**Table 1**

| **open issue** | **severity** | **status** | **most probable root cause** | **second-most probable root cause** | **third-most probable root cause** |
|---|---|---|---|---|---|
| open issue 1 | severity 1 | next actions | details including probability and severity I_1,1 | details including probability and severity I_1,2 | details including probability and severity I_1,3 |
| open issue 2 | severity 2 | next actions | details including probability and severity I_2,1 | details including probability and severity I_2,2 | details including probability and severity I_2,3 |
| open issue 3 | severity 3 | next actions | details including probability and severity I_3,1 | details including probability and severity I_3,2 | details including probability and severity I_3,3 |
| open issue 4 | severity 4 | next actions | details including probability and severity I_4,1 | details including probability and severity I_4,2 | details including probability and severity I_4,3 |
| open issue 5 | severity 5 | next actions | details including probability and severity I_5,1 | details including probability and severity I_5,2 | details including probability and severity I_5,3 |

Table 1 shows a main window of the UI showing a list of open issues, ordered by decreasing severity. The third column gives the status of the open issue, showing for example how long it has been open, whether or not there are already next actions planned, in case an appointment is required for a remote login. The fourth, fifth and sixth columns give for each open issue details on the three most probable root causes. These details include the severity and probability of the root cause, as well as other possible details such as the effort required to determine whether this is the actual root cause.

By clicking on an open issues in the main window, further details are shown in a next window that is dedicated to the specific open issue, showing a longer list of possible root causes and further details on how to further work on the open issue. This second window gives further details on how long the issue has been open and the actions that have already been performed to resolve it, an overview of the actions that could be carried out, split up into the actions that do not require remote login and actions that do require remote login. Furthermore, an estimated duration of each of the actions is given. If appropriate, also appointments are shown for a remote login at a moment that is appropriate for the hospital. This is shown in Table 2.

**Table 2**

| **open issue i** |
|---|
| history of open issue including list of actions already performed |
| possible appointment for remote login |
| details on root cause I_i,1 |
| details on root cause I_i,2 |
| details on root cause I_i,3 |
| details on root cause ... |

Table 2 shows an example of a new window, showing, for a selected open issue, amongst others, the history of the open issue including the time that it was raised and the history of actions that have already been performed to resolve it. The window also includes possible appointments that have already been made with the hospital to carry out a next action that requires remote login at a moment that is convenient to the hospital. In addition, the window gives details of the complete list of root causes that still have a positive probability, given the already performed actions.

By automatically tracking the progress of each of the open issues, one can automatically generate alerts whenever issues have not made any progress during a given time interval. These alerts can be set relative to the moment the issue was raised or the alerts can be set relative to the last moment there was any progress for the given issue. Furthermore, the estimated severity *s*(*i*) of an issue *i*, as defined by one of the above mentioned alternatives, can be used to determine whether or not such an alert should be raised. An alert can be generated whenever a weighted sum of time passed, and severity reaches a certain threshold. Alternatively, the size of the time durations (either relative to the moment the issue was detected or relative to the last progress) can be chosen to depend on the estimated severity *s*(*i*) of the issue.

Alternatively, a service engineer can set a timer to generate an alert, whenever a given parameter has exceeded a manually chosen threshold. For example, if the number of files in a given input queue has raised an alert, then a possible action for the resulting open issue can be to temporarily set a new higher threshold such that a higher priority alert will be raised whenever this higher threshold is also exceeded. Given their personal experience, service engineers may want to see whether the queue is simply experiencing a temporarily high load that will resolve without human intervention after a short time or not.

Tracking of a proactive monitoring team can more easily allow assisting third-party service providers without giving away too much of knowledge on how to solve these issues. The third-party service providers would not be given the details of the resolution trees and the estimated severities and probabilities. They would only be given advice on what to do next. Given the diversity of the issues that might be open simultaneously, it would be very difficult for them to extract useful values for these severities and probabilities from these recommendations.

The servicing support system **100** is applicable to various types of medical devices for which remote login is important to determine the root cause of possible issues. Remote login may not always be desirable on arbitrary moments in time, taking into account patient safety. This includes medical imaging systems as well as PACS. Furthermore, the servicing support system **100** might be applicable for other fields of application where unplanned downtime should be avoided and performing additional checks may interfere with normal business operations.

A non-transitory storage medium includes any medium for storing or transmitting information in a form readable by a machine (e.g., a computer). For instance, a machine-readable medium includes read only memory ("ROM"), solid state drive (SSD), flash memory, or other electronic storage medium; a hard disk drive, RAID array, or other magnetic disk storage media; an optical disk or other optical storage media; or so forth.

The methods illustrated throughout the specification, may be implemented as instructions stored on a non-transitory storage medium and read and executed by a computer or other electronic processor.

The disclosure has been described with reference to the preferred embodiments. Modifications and alterations may occur to others upon reading and understanding the preceding detailed description. It is intended that the exemplary embodiment be construed as including all such modifications and alterations insofar as they come within the scope of the appended claims.

## Claims

1. A non-transitory computer readable medium (**107, 127**) storing:
a resolution tree (**130**) comprising data related to resolution of a maintenance issue of a medical imaging device (**120**), the resolution tree including leaf nodes (**132**) corresponding to possible root causes of the maintenance issue, wherein each leaf node includes a probability of the corresponding root cause and a severity of the corresponding root cause; and
instructions readable and executable by at least one electronic processor (**101, 113**) to:
receive data related to results of a completed service action performed to diagnose the maintenance issue;
update the resolution tree with the received data including updating the probabilities of the root causes of the corresponding leaf nodes;
identify one or more root causes to be investigated based at least on the updated probabilities of the root causes of the corresponding leaf nodes and the severities of the corresponding root causes;
display, on a display device (**105**) of a remote electronic processing device (**102**) operable by a service engineer (SE), the one or more root causes to be investigated and the updated probabilities of the respective one or more root causes to be investigated; and
i) repeat the receive, update, and display operations for each successive completed service action to update the displayed one or more possible root causes to be investigated and the probabilities of the respective one or more root causes to be investigated;
or
ii) receive a schedule (**138**) of scheduled service actions from a scheduler; and
output a reminder to perform or schedule one or more of the service actions to be performed that are not scheduled service actions.

2. The non-transitory computer readable medium (**107, 127**) of claim 1, wherein the display operation further displays the severities of the respective one or more root causes to be investigated.

3. The non-transitory computer readable medium (**107, 127**) of claim 1, wherein the resolution tree (**130**) further includes:
a root node (**131**) corresponding to the maintenance issue, and
a plurality of intermediate nodes (**133**) each representing service actions which can be carried out attempting to resolve the maintenance issue.

4. The non-transitory computer readable medium (**107, 127**) of claim 3, wherein the instructions further identify one or more service actions to be performed based on the one or more root causes to be investigated and the intermediate nodes (**133**).

5. The non-transitory computer readable medium (**107, 127**) of claim 4, wherein the instructions further include:
receive a schedule (**138**) of scheduled service actions from a scheduler; and
output a reminder to perform or schedule one or more of the service actions to be performed that are not scheduled service actions.

6. The non-transitory computer readable medium (**107, 127**) of any one of claims1 - 5, wherein updating the resolution tree (**130**) with the received data includes:
computing updated severity values based on historical data of historical service actions that were successful in resolving previous maintenance issues; and
updating the resolution tree with the computed updated severity values.

7. The non-transitory computer readable medium (**107, 127**) of claim 6, wherein computing the updated severity values includes:
computing an aggregate severity for the maintenance issue as a sum over all leaf nodes (**132**) as the product of the computed probability and severity values; and
issuing an alert regarding the maintenance issue if the aggregate severity exceeds a threshold.

8. The non-transitory computer readable medium (**107, 127**) of either one of claims 6 and 7, wherein the display device (**105**) is configured to display a first UI (**140**) is configured to show the computed aggregate severity for the maintenance issue.

9. The non-transitory computer readable medium (**107,127**) of either one of claims 7 and 8, wherein the display device (**105**) is configured to display a second UI (**142**) configured to show information related to a specific root cause of the maintenance issue.

10. The non-transitory computer readable medium (**107, 127**) of claim 9, wherein the instructions further include:
displaying the second UI (**142**) upon receiving a user input indicative of a selection of a portion of the first UI (**140**).

11. A method (**200**) comprising:
providing a resolution tree including leaf nodes (**132**) corresponding to possible root causes of a maintenance issue, wherein each leaf node includes a probability and a severity of the corresponding root cause;
receiving data related to results of a completed service action performed to diagnose the maintenance issue;
updating the resolution tree (**130**) with the received data including updating the probabilities and severities of the root causes of the corresponding leaf nodes;
identifying one or more root causes to be investigated based at least on the updated probabilities and updated severities of the root causes of the corresponding leaf nodes; and
displaying, on a display device (**105**), the one or more root causes to be investigated and the updated probabilities and updated severities of the respective one or more root causes to be investigated.

## Patentansprüche

1. Nichtflüchtiges computerlesbares Medium (107, 127), das Folgendes speichert:
einen Auflösungsbaum (130), der Daten umfasst, die sich auf die Auflösung eines Wartungsproblems einer medizinischen Bildgebungsvorrichtung (120) beziehen, wobei der Auflösungsbaum Blattknoten (132) beinhaltet, die möglichen Grundursachen des Wartungsproblems entsprechen, wobei jeder Blattknoten eine Wahrscheinlichkeit der entsprechenden Grundursache und einen Schweregrad der entsprechenden Grundursache beinhaltet; und
Anweisungen, die durch mindestens einen elektronischen Prozessor (101, 113) lesbar und ausführbar sind, um Folgendes zu bewirken:
Empfangen von Daten in Bezug auf die Ergebnisse einer abgeschlossenen Serviceaktion, die zur Diagnose des Wartungsproblems durchgeführt wurde;
Aktualisieren des Auflösungsbaums mit den empfangenen Daten, einschließlich Aktualisieren der Wahrscheinlichkeiten der Grundursachen der entsprechenden Blattknoten;
Identifizieren einer oder mehrerer zu untersuchender Grundursachen basierend zumindest auf den aktualisierten Wahrscheinlichkeiten der Grundursachen der entsprechenden Blattknoten und den Schweregraden der entsprechenden Grundursachen;
Anzeigen, auf einer Anzeigevorrichtung (105) einer entfernten elektronischen Verarbeitungsvorrichtung (102), die durch einen Servicetechniker (SE) betreibbar ist, der einen oder der mehreren Grundursachen, die untersucht werden sollen, und der aktualisierten Wahrscheinlichkeiten der jeweiligen einen oder der mehreren Grundursachen, die untersucht werden sollen; und
i) Wiederholen der Empfangs-, Aktualisierungs- und Anzeigeoperationen für jede aufeinanderfolgende abgeschlossene Serviceaktion, um die angezeigten einen oder mehreren möglichen zu untersuchenden Grundursachen und die Wahrscheinlichkeiten der jeweiligen einen oder mehreren zu untersuchenden Grundursachen zu aktualisieren;
oder
ii) Empfangen eines Zeitplans (138) von geplanten Serviceaktionen von einem Scheduler; und
Ausgeben einer Mahnung zum Durchführen oder Planen einer oder mehrerer der durchzuführenden Serviceaktionen, bei denen es sich nicht um geplante Serviceaktionen handelt.

2. Nichtflüchtiges computerlesbares Medium (107, 127) nach Anspruch 1, wobei die Anzeigeoperation ferner die Schweregrade der jeweiligen einen oder mehreren zu untersuchenden Grundursachen anzeigt.

3. Nichtflüchtiges computerlesbares Medium (107, 127) nach Anspruch 1, wobei der Auflösungsbaum (130) ferner Folgendes beinhaltet:
einen Wurzelknoten (131), der dem Wartungsproblem entspricht, und
eine Vielzahl von Zwischenknoten (133), die jeweils Serviceaktionen repräsentieren, die ausgeführt werden können, um das Wartungsproblem zu lösen.

4. Nichtflüchtiges computerlesbares Medium (107, 127) nach Anspruch 3, wobei die Anweisungen ferner eine oder mehrere durchzuführende Serviceaktionen basierend auf der einen oder den mehreren zu untersuchenden Grundursachen und den Zwischenknoten (133) identifizieren.

5. Nichtflüchtiges computerlesbares Medium (107, 127) nach Anspruch 4, wobei die Anweisungen ferner Folgendes beinhalten:
Empfangen eines Zeitplans (138) von geplanten Serviceaktionen von einem Scheduler; und
Ausgeben einer Mahnung zum Durchführen oder Planen einer oder mehrerer der durchzuführenden Serviceaktionen, bei denen es sich nicht um geplante Serviceaktionen handelt.

6. Nichtflüchtiges computerlesbares Medium (107, 127) nach einem der Ansprüche 1 - 5, wobei das Aktualisieren des Auflösungsbaums (130) mit den empfangenen Daten Folgendes beinhaltet:
Berechnen aktualisierter Schweregrade basierend auf historischen Daten historischer Serviceaktionen, die bei der Lösung früherer Wartungsprobleme erfolgreich waren; und
Aktualisieren des Auflösungsbaums mit den berechneten aktualisierten Schweregradwerten.

7. Nichtflüchtiges computerlesbares Medium (107, 127) nach Anspruch 6, wobei das Berechnen der aktualisierten Schweregradwerte Folgendes beinhaltet:
Berechnen eines aggregierten Schweregrads für das Wartungsproblem als eine Summe über alle Blattknoten (132) als das Produkt der berechneten Wahrscheinlichkeits- und Schweregradwerte; und
Ausgeben einer Warnung bezüglich des Wartungsproblems, wenn der Gesamtschweregrad einen Schwellenwert überschreitet.

8. Nichtflüchtiges computerlesbares Medium (107, 127) nach einem der Ansprüche 6 und 7, wobei die Anzeigevorrichtung (105) dazu ausgelegt ist, eine erste UI (140) anzuzeigen, die dazu ausgelegt ist, den berechneten aggregierten Schweregrad für das Wartungsproblem anzuzeigen.

9. Nichtflüchtiges computerlesbares Medium (107, 127) nach einem der Ansprüche 7 und 8, wobei die Anzeigevorrichtung (105) dazu ausgelegt ist, eine zweite UI (142) anzuzeigen, die dazu ausgelegt ist, Informationen bezüglich einer spezifischen Grundursache des Wartungsproblems anzuzeigen.

10. Nichtflüchtiges computerlesbares Medium (107, 127) nach Anspruch 9, wobei die Anweisungen ferner Folgendes beinhalten:
Anzeigen der zweiten UI (142) beim Empfangen einer Benutzereingabe, die eine Auswahl eines Teils der ersten UI (140) angibt.

11. Verfahren (200), das Folgendes umfasst:
Bereitstellen eines Auflösungsbaums, der Blattknoten (132) beinhaltet, die möglichen Grundursachen eines Wartungsproblems entsprechen, wobei jeder Blattknoten eine Wahrscheinlichkeit und eine Schwere der entsprechenden Grundursache beinhaltet;
Empfangen von Daten im Zusammenhang mit den Ergebnissen einer abgeschlossenen Serviceaktion, die zur Diagnose des Wartungsproblems durchgeführt wurde;
Aktualisieren des Auflösungsbaums (130) mit den empfangenen Daten, einschließlich Aktualisieren der Wahrscheinlichkeiten und Schweregrade der Grundursachen der entsprechenden Blattknoten;
Identifizieren einer oder mehrerer zu untersuchender Grundursachen basierend zumindest auf den aktualisierten Wahrscheinlichkeiten und aktualisierten Schweregraden der Grundursachen der entsprechenden Blattknoten; und
Anzeigen, auf einer Anzeigevorrichtung (105), der einen oder der mehreren Grundursachen, die untersucht werden sollen, und der aktualisierten Wahrscheinlichkeiten und aktualisierten Schweregrade der jeweiligen einen oder der mehreren Grundursachen, die untersucht werden sollen.

## Revendications

1. Support non transitoire lisible par ordinateur (107, 127) stockant :
un arbre de résolution (130) comprenant des données relatives à la résolution d'un problème de maintenance d'un dispositif d'imagerie médicale (120), l'arbre de résolution comportant des nœuds feuilles (132) correspondant à des causes racines possibles du problème de maintenance, chaque nœud feuille comportant une probabilité de la cause racine correspondante et un niveau de gravité de la cause racine correspondante ; et
des instructions lisibles et exécutables par au moins un processeur électronique (101, 113) pour :
recevoir des données relatives à des résultats d'une action de service effectuée pour diagnostiquer le problème de maintenance ;
mettre à jour l'arbre de résolution avec les données reçues, incluant la mise à jour des probabilités des causes racines des nœuds feuilles correspondants ;
identifier une ou plusieurs causes racines à étudier sur la base au moins des probabilités mises à jour des causes racines des nœuds feuilles correspondants et des niveaux de gravité des causes racines correspondantes ;
afficher, sur un dispositif d'affichage (105) d'un dispositif de traitement électronique à distance (102) exploitable par un ingénieur de service (SE), la ou les causes racines à examiner et les probabilités mises à jour de la ou des causes racines respectives à examiner ; et
i) répéter les opérations de réception, de mise à jour et d'affichage pour chaque action de service terminée successive afin de mettre à jour la ou les causes racines possibles affichées à examiner et les probabilités de la ou des causes racines respectives à examiner ;
ou
ii) recevoir un calendrier (138) d'actions de service planifiées en provenance d'un planificateur ; et
sortir un rappel invitant à effectuer ou planifier une ou plusieurs des actions de service à effectuer qui ne sont pas des actions de service planifiées.

2. Support non transitoire lisible par ordinateur (107, 127) selon la revendication 1, dans lequel l'opération d'affichage affiche en outre les niveaux de gravité de la ou des causes racines respectives à examiner.

3. Support non transitoire lisible par ordinateur (107, 127) selon la revendication 1, dans lequel l'arbre de résolution (130) comporte en outre :
un nœud racine (131) correspondant au problème de maintenance, et
une pluralité de nœuds intermédiaires (133) représentant chacun des actions de service qui peuvent être menées en essayant de résoudre le problème de maintenance.

4. Support non transitoire lisible par ordinateur (107, 127) selon la revendication 3, dans lequel les instructions identifient en outre une ou plusieurs actions de service à effectuer sur la base de la ou des causes racines à examiner et des nœuds intermédiaires (133).

5. Support non transitoire lisible par ordinateur (107, 127) selon la revendication 4, dans lequel les instructions consistent en outre à :
recevoir un calendrier (138) d'actions de service planifiées en provenance d'un planificateur ; et
sortir un rappel invitant à effectuer ou planifier une ou plusieurs des actions de service à effectuer qui ne sont pas des actions de service planifiées.

6. Support non transitoire lisible par ordinateur (107, 127) selon l'une quelconque des revendications 1 à 5, dans lequel la mise à jour de l'arbre de résolution (130) avec les données reçues consiste à :
calculer des valeurs de gravité mises à jour sur la base de données historiques d'actions de service historiques qui ont réussi à résoudre des problèmes de maintenance précédents ; et
mettre à jour l'arbre de résolution avec les valeurs de gravité mises à jour calculées.

7. Support non transitoire lisible par ordinateur (107, 127) selon la revendication 6, dans lequel le calcul des valeurs de gravité mises à jour consiste à :
calculer un niveau de gravité agrégé pour le problème de maintenance en tant que somme, sur tous les nœuds feuilles (132), du produit des valeurs de probabilité et de gravité calculées ; et
émettre une alerte concernant le problème de maintenance si le niveau de gravité globale dépasse un seuil.

8. Support non transitoire lisible par ordinateur (107, 127) selon l'une quelconque des revendications 6 et 7, dans lequel le dispositif d'affichage (105) est configuré pour afficher une première interface utilisateur (140) qui est configurée pour montrer le niveau de gravité agrégée calculée pour le problème de maintenance.

9. Support non transitoire lisible par ordinateur (107, 127) selon l'une ou l'autre des revendications 7 et 8, dans lequel le dispositif d'affichage (105) est configuré pour afficher une seconde interface utilisateur (142) configurée pour afficher des informations relatives à une cause racine spécifique du problème de maintenance.

10. Support non transitoire lisible par ordinateur (107, 127) selon la revendication 9, dans lequel les instructions consistent en outre à :
afficher la seconde interface utilisateur (142) lors de la réception d'une entrée utilisateur indiquant une sélection d'une partie de la première interface utilisateur (140).

11. Procédé (200) comprenant :
la fourniture d'un arbre de résolution incluant des nœuds feuilles (132) correspondant à des causes racines possibles d'un problème de maintenance, chaque nœud feuille comportant une probabilité et un niveau de gravité de la cause racine correspondante ;
la réception de données relatives à des résultats d'une action de service terminée pour diagnostiquer le problème de maintenance ;
la mise à jour de l'arbre de résolution (130) avec les données reçues, incluant la mise à jour des probabilités et des niveaux de gravité des causes racines des nœuds feuilles correspondants ;
l'identification d'une ou plusieurs causes racines à examiner sur la base au moins des probabilités mises à jour et des niveaux de gravité mis à jour des causes racines des nœuds feuilles correspondants ; et
l'affichage, sur un dispositif d'affichage (105), de la ou des causes racines à examiner et des probabilités et des niveaux de gravité mis à jour de la ou des causes racines respectives à examiner.
